# EUROPEAN PATENT SPECIFICATION

(11) **EP 1 011 764 B1**
(45) Date of publication and mention of the grant of the patent: **04.02.2004**
(21) Application number: 97912596.0
(22) Date of filing: 28.10.1997
(51) Int. Cl.: A61M 5/32

(54) **I.V. CATHETER WITH NEEDLE GUARD**
INTRAVENÖSER KATHETER MIT NADELSCHUTZ
CATHETER INTRAVEINEUX AVEC GAINE DE PROTECTION DE L'AIGUILLE

(30) Priority: 04.11.1996 SE 9604021
(43) Date of publication of application: 28.06.2000
(73) Proprietor: Becton Dickinson and Company, Franklin Lakes, New Jersey 07417-1880 (US)
(72) Inventor: AXELSSON, Robert, S-560 28 Lekeryd (SE)
(74) Representative: Ruffles, Graham Keith
(86) International application number: PCT/SE1997/001788
(87) International publication number: WO 1998/019725

(56) References cited:
- EP-A- 0 645 159

## Description

The present invention relates to a needle guard for a so called I.V. Catheter having a catheter housing, a catheter tube and an insertion needle. The insertion needle can be passed through a central passage in the needle guard into the catheter tube to a position in which the distal tip of the insertion needle is exposed and located outside the catheter tube and by means of a blocking device encases the needle tip within the needle guard at withdrawal of the insertion needle from the catheter housing together with the needle guard.

The task of the needle guard at the withdrawal of the insertion needle from the catheter housing, after the catheter tube having been entered into a blood vessel is to prevent accidential injuries by the insertion needle when separating the needle from the catheter housing since this may lead to serious infections if the insertion needle should be infected. The needle guard can also prevent reuse of the insertion needle that could be a disaster if the insertion needle is infected and to simplify destruction of the insertion needle by bending it and thus making it unfit for use without any risk of pricking oneself.

In EP 645,159-A, a catheter stick protector is described having a metal flap clip which snaps down into place to prevent return of the needle through the protector device once withdrawn. The device is small and received over the needle to provide an interface between the needle hub and the hub of a catheter like product. A sleeve of hydrophobic or fluid impermeable material is provided and attached at one end to the needle hub and at a second end to the tip protector. The length of the material is selected to hold the tip protector in a position just extending beyond the tip of the needle of a catheter inserter.

Since the price for I.V. catheters today is heavily forced down one runs the risk that an I.V. catheter cannot bear the additional cost connected with expensive technical solutions of needle guards, which prevents a more general use of such a so called safety catheter while the present invention can be produced at a very low cost.

The purpose of the present invention is to provide a device of the kind referred to above which comprises a needle guard of a construction which allows manufacture of the needle guard in just one injection molding operation, possibly together with the rear portion of the insertion needle in plastic material which will result in a low cosi product and also allows an automatic soft and jerkless handling when encasing the needle tip into the needle guard at withdrawal from the catheter housing.

The mentioned purpose is achieved by the device according to the invention having obtained the charactenstics of claim 1

The invention will be described in more detail below reference being made to the accompanying drawing in which

Fig1 is a schematic cross sectional view of an illustrative embodiment of an I.V. Catheter according to the invention before the insertion needle being inserted.

Fig 2 is a corresponding view under withdrawal of the insertion needle,

Fig 3 is a corresponding view with the insertion needle as well as the needle guard completely withdrawn and seperated from the catheter housing
The drawing shows a conventional I.V Catheter where the conical catheter housing 7 is constructed to form a connection socket in a Luer lock mechanism 5 for connection of a syringe an inlusion hose or the like

In the bottom portion a catheter tube 1 in the shape of a thin plastic hose for insertion into a blood vessel of a patient, and a pair of wings 11 project from the catheter housing at opposite sides thereof, at which the catheter housing can be attached to the patient by means of for example plaster or the like. The I.V Catheter includes also as a separate part thereof an insertion needle 9 with a needle tip 2 at one end the other end being connected to a rear portion 3 of plastic matenal.

When applying the I V Catheter the purpose of the insertion needle is to penetrate a blood vessel of a patient and to introduce the catheter tube 1 into the blood vessel the insertion needle 9 shiffening the catheter tube. After insertion the insertion needle is withdrawn while the catheter tube is left in the hlood vessel This is the normal way of applying an I V Catheter

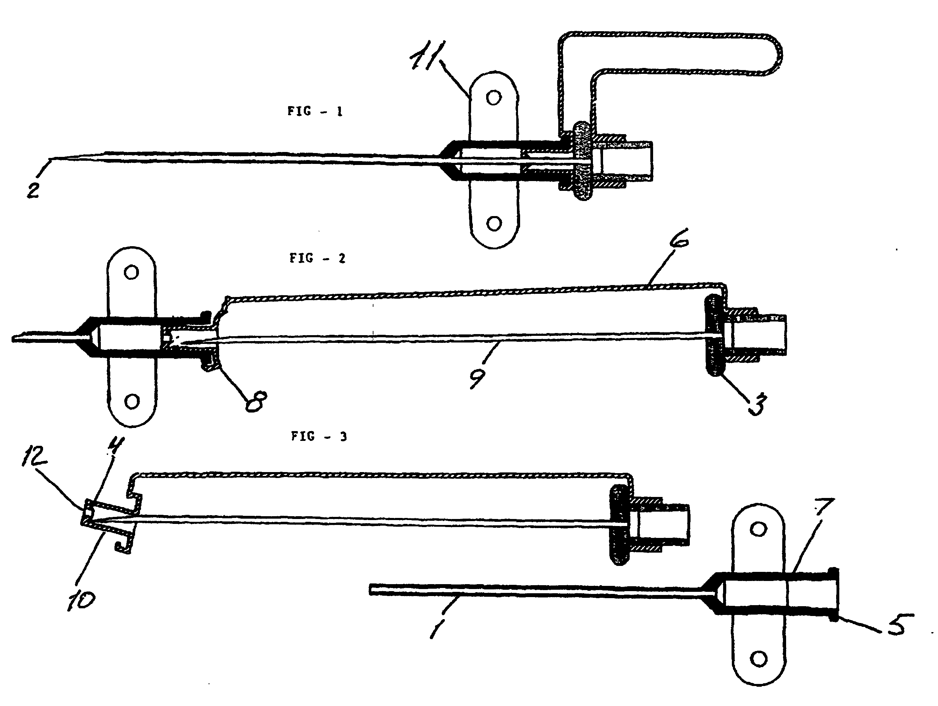

The needle guard 10 is positioned and is attached to the Luer lock mechanism by means of a snap device 8 as is more closely illustrated by the drawing, figs 1 - 2 while a cord 6 of the needle guard either is fastened to the rear portion 3 of the insertion needle or is included in the rear portion from the very beginning i e. at the manufacture of the rear portion and needle guard / cord. thus forming just one unit in injection molding The needle guard has a central opening 12 the diameter of which allows easy introduction of the insertion needle therethrough at easy slip fit

The needle guard shall be made of plastic material that must not be rigid but Shall have a certain elasticity in order to admit an extension of the cord 6 when it is stretched then to spring back when the force generated by the snap device ceases, to a blocking position in the needle guard.

After the insertion needle having been introduced into a blood vessel of the patient in the manner previously described for the introduction of the catheter tube 1 into the blood vessel, the insertion needle 9 shall be withdrawn from the cathteter housing 7 while the catheter tube 1 remains in the blood vessel When the needle tip 2 at withdrawal of the insertion needle passes the central passage 12 the cord 6 will be extended and the shap device 8 will be disengaged the needle tip 2 of the insertion needle being encased and blocked in the needle guard 10 The needle tip is safety retained and locked in the needle guard, since it cannot be ejected because the needle tip engages the shoulder surface 4, and it cannot be withdrawn because the cord cannot be extended to the extent that the needle tip is allowed to leave the needle guard. The tip of the insertion needle is safely protected inside the needle guard without risk of nursing personell handling the I V. Catheter pricking themselves on the needle tip. There is also no risk of this happining when the insertion needle after withdrawel from the catheter housing is destroyed by bending

## Claims

1. I.V. Catheter including a catheter housing (7), a catheter tube (1), an insertion needle (9) and a needle guard (10) inside the catheter housing the insertion needle being insertable into the catheter tube through a central passage (12) in the needle guard to a position in which the tip (2) of the insertion needle is located outside the catheter tube and via a blocking device (4, 6) encases the needle tip within the needle guard at withdrawal of the insertion needle (9) from the catheter housing together with the needle guard (10), **characterized in that** the needle guard is attached by means of a snap device (8) to a Luer lock mechanism (5) of the catheter housing for retaining the needle guard in the catheter housing (7), and via an elastic cord (6) to the insertion - needle-passed Into the catheter housing encloses the needle tip (2) at withdrawel of the insertion needle from the catheter housing when the cord is being extended the snap device (8) being disengaged from the catheter housing and the insertion needle (9) together with the needle guard (10) being separated from the catheter housing (7).

2. Device according to claim 1, **characterized In that** inside the needle guard (10) there are provided as locking device inclined surfaces (4) retaining and locking the needle tip.

3. Device according to claims 1-2, **characterized in in that** a shoulder in the needle guard (10) guides the insertion needle (9) at withdrawel from the catheter housing (7) in order to make sure that the needle tip (2) will arrive at one and the same locking position in the needle guard.

4. Device according to claims 1-3, **characterized In that** needle guard / cord (10, 6) are of the same plastic material as that of the rear portion (3) of the insertion needle and thus is incorporated as an integral single unit manufacture of the rear portion (3) the insertion needle including also the needle guard / cord (10, 6) and being performed in just one operation.

## Patentansprüche

1. Intravenöser Katheter mit einem Kathetergehäuse (7), einer Katheterröhre (1), einer Einführnadel (9) und einem Nadelschutz (10) innerhalb des Kathetergehäuses, wobei die Einführnadel in die Katheterröhre durch einen zentralen Kanal (12) in dem Nadelschutz zu einer Position geführt werden kann, in der sich die Spitze (2) der Einführnadel außerhalb der Katheterröhre befindet, und über eine Blockiervorrichtung (4, 6) die Nadelspitze beim Herausziehen der Einführnadel (9) aus dem Kathetergehäuse zusammen mit dem Nadelschutz (10) innerhalb des Nadelschutzes umschließt, **dadurch gekennzeichnet, dass** der Nadelschutz mit Hilfe einer Schnappvorrichtung (8) an einem Lüerschen Mechanismus (5) des Kathetergehäuses befestigt ist, um den Nadelschutz in dem Kathetergehäuse (7) festzuhalten und, über eine Elastikschnur (6) zu der in das Kathetergehäuse geführten Einführnadel, die Nadelspitze (2) beim Herausziehen der Einführnadel aus dem Kathetergehäuse umschließt, wenn die Schnur verlängert wird, wobei die Schnappvorrichtung (8) von dem Kathetergehäuse gelöst und die Einführnadel (9) zusammen mit dem Nadelschutz (10) von dem Kathetergehäuse (7) getrennt wird.

2. Vorrichtung nach Anspruch 1, **dadurch gekennzeichnet, dass** innerhalb des Nadelschutzes (10) abgeschrägte Flächen (4) als Verriegelungsvorrichtung vorgesehen sind, die die Nadelspitze festhalten und festklemmen.

3. Vorrichtung nach den Ansprüchen 1-2, **dadurch gekennzeichnet, dass** ein Ansatz im Nadelschutz (10) die Einführnadel (9) beim Herausziehen aus dem Kathetergehäuse (7) führt, um sicherzustellen, dass die Nadelspitze (2) an ein und derselben Verriegelungsposition im Nadelschutz ankommt.

4. Vorrichtung nach den Ansprüchen 1-3, **dadurch gekennzeichnet, dass** Nadelschutz/Schnur (10, 6) aus dem gleichen Kunststoffmaterial wie der hintere Teil (3) der Einführnadel bestehen, so dass der hintere Teil (3) der Einführnadel sowie der Nadelschutz/die Schnur (10, 6) in einem einzigen Herstellungsvorgang einstückig ausgebildet werden können.

## Revendications

1. Cathéter intraveineux incluant un logement de cathéter (7), un tube de cathéter (1), une aiguille d'insertion (9) et une gaine de protection d'aiguille (10) à l'intérieur du logement de cathéter, l'aiguille d'insertion pouvant être insérée dans le tube de cathéter à travers un passage central (12) dans la gaine de protection d'aiguille pour atteindre une position dans laquelle la pointe (2) de l'aiguille d'insertion est située à l'extérieur du tube de cathéter, et enferme la pointe de l'aiguille à l'intérieur de la gaine de protection d'aiguille, par le biais d'un dispositif de blocage (4, 6), au moment du retrait de l'aiguille d'insertion (9) du logement de cathéter avec la gaine de protection d'aiguille (10), **caractérisé par le fait que** la gaine de protection d'aiguille est fixée, au moyen d'un dispositif à déclic (8), à un mécanisme de verrouillage Luer Lock (5) du logement de cathéter afin de retenir la gaine de protection d'aiguille dans le logement de cathéter (7) et, par le biais d'un cordon élastique (6) relié à l'aiguille d'insertion passée dans le logement de cathéter, entoure la pointe (2) de l'aiguille au moment du retrait de l'aiguille d'insertion du logement de cathéter lorsque le cordon est étendu, le dispositif à déclic (8) étant déclenché d'avec le logement de cathéter et l'aiguille d'insertion (9), avec la gaine de protection d'aiguille (10), étant séparée du logement de cathéter (7).

2. Dispositif selon la revendication 1, **caractérisé par le fait que** l'intérieur de la gaine de protection d'aiguille (10) est pourvu, en tant que dispositif de verrouillage, de surfaces inclinées (4) qui retiennent et verrouillent la pointe de l'aiguille.

3. Dispositif selon les revendications 1-2, **caractérisé par le fait qu'**un épaulement dans la gaine de protection d'aiguille (10) guide l'aiguille d'insertion (9) au moment de son retrait du logement de cathéter (7) de manière à s'assurer que la pointe (2) de l'aiguille adoptera une seule et unique position de verrouillage dans la gaine de protection d'aiguille.

4. Dispositif selon les revendications 1-3, **caractérisé par le fait que** la gaine de protection d'aiguille / le cordon (10, 6) sont formés de la même matière plastique que la partie arrière (3) de l'aiguille d'insertion et sont incorporés ainsi en tant qu'unité de fabrication unique intégrale de la partie arrière (3), l'aiguille d'insertion incluant également la gaine de protection d'aiguille / le cordon (10, 6) et étant exécutée en une seule opération.
